# EUROPEAN PATENT SPECIFICATION

(11) **EP 1 061 938 B1**
(45) Date of publication and mention of the grant of the patent: **14.11.2001**
(21) Application number: 99906246.6
(22) Date of filing: 18.02.1999
(51) Int. Cl.: A61K 38/16, A61K 38/17, A61K 9/00

(54) **ORAL COMPOSITIONS AT LOW DOSAGE OF CYTOTOXIC PROTEINS**
ORALE ZUSAMMENSETZUNGEN MIT NIEDRIGDOSIERTEN ZYTOTOXISCHEN PROTEINEN
COMPOSITIONS S'ADMINISTRANT PAR VOIE ORALE A FAIBLE TENEUR EN PROTEINES CYTOTOXIQUES

(30) Priority: 24.02.1998 IT MI980356
(43) Date of publication of application: 27.12.2000
(73) Proprietor: ZETESIS S.P.A., 20122 Milano (IT)
(72) Inventor: BARTORELLI, Alberto, I-20145 Milano (IT); SANTI, Cesare, I-20122 Milano (IT)
(74) Representative: Minoja, Fabrizio, Dr.
(86) International application number: EP9901068
(87) International publication number: WO9943340

(56) References cited:
- WO-A-92/10197
- WO-A-96/02567
- GB-A- 989 826

## Description

The present invention relates to pharmaceutical compositions for oral and sublingual administration containing proteins extractable from mammalian liver.

Such proteins include the proteins referred to as UK101 and UK114 and described in WO 92/10197 and WO 96/02567 as well as ubiquitin, contained in the UK101 extract.

It has been observed that the subcutaneous administration of UK101 and UK114 induces a clear cytotoxicity in serum of both healthy subjects and in tumor carriers subjects.

The main responsible for this effect is the 14Kd protein (UK114) contained in the protein extract UK101. The UK114 amino acidic sequence has been described in FEBS Let. 393, 147-150, 1996.

At present, clinical experimentations to verify the UK101 and UK114 therapeutic efficacy are in progress. To this order, patients suffering from colon and breast carcinoma are treated with UK101 and UK114 subcutaneous injections, at dosages ranging from 1 to 10 mg/week.

The protein nature of the active principle obviously forces the parenteral route

In fact, at present it is not known the possibility to administer proteins orally, due to their high metabolic instability.

To avert this inconvenience, it has been suggested several answers such as the use of suitable carriers or the encapsulation in liposomes, but until now the results have been unfavourable.

Now it has been found that it is possible to administer UK114 and UK101 or ubiquitin orally at low dosages, preferably for sublingual administration, thus inducting a seric cytotoxicity comparable to or higher than that obtainable for subcutaneous administration.

The oral/sublingual route also shows clear advantages in practicality and safety terms.

The invention therefore provides pharmaceutical compositions for UK101 and UK114 oral and/or sublingual administration.

Suitable administration forms include, for example, aqueous suspensions to administer in drops, granules or sublingual tablets by a quickly disintegration, effervescent or chewable tablets or its equivalent forms.

The compositions of the invention can be prepared using conventional techniques and excipients, widely known in the pharmaceutical field.

The UK101 and UK114 unitary dosages can range from 10 x 10⁻⁴ to 10 x 10⁻¹⁵g.

In the case of solutions to administrate in drops, the concentration of the active principle can range from 10⁻⁵ to 10⁻¹⁰ M. The administration of 10-15 drops a day proved to be sufficient to induce cytotoxicity in the patient serum, which can be evidenced on carcinoma cells Jurkat and Kato III according to standard protocols.

### EXAMPLE

17 patients suffering from tumors in advanced phase (8 sarcoma, 4 breast carcinoma, 2 pancreas carcinoma and 3 colon carcinoma) have been treated with 5-20 drops/day of an 1% hydroalcoholic solution of ethanol in a UK101 concentration of 10⁻⁶ M.

The treatment continued for 30 consecutive days, involved in 70% of the cases an improvement in the subjective conditions of the patients, particularly a decrease in the painful symptomatology, a decrease in the tumor mass in 20% of the cases, joined to a cytotoxicity visible in the patients serum on cell lines Jurkat and Kato III.

## Claims

1. Pharmaceutical compositions containing as active principle a protein selected from ubiquitin, UK114 and UK 101 in form of granules or sublingual tablets.

2. The use of ubiquitin, UK 114 and UK 101 for the preparation of a medicament for oral administration.

## Patentansprüche

1. Pharmazeutische Zusammensetzungen, die als Wirkstoff ein Protein, das aus Ubiquitin, UK 114 und UK 101 ausgewählt ist, enthalten, in Form von Granulat oder sublingualen Tabletten.

2. Verwendung von Ubiquitin, UK 114 und UK 101 zur Herstellung eines Arzneimittels zur oralen Verabreichung.

## Revendications

1. Compositions pharmaceutiques contenant, à titre de principe actif, une protéine choisie entre l'ubiquitine, la UK 114 et la UK 101 sous forme de granules ou de comprimés sublinguaux.

2. Utilisation de l'ubiquitine, de l'UK 114 et de l'UK 101 pour la préparation d'un médicament pour l'administration par voie orale.
